# FASCICULE DE BREVET EUROPEEN

(11) **EP 2 732 275 B1**
(45) Date de publication et mention de la délivrance du brevet: **31.07.2019**
(21) Numéro de dépôt: 12729917.0
(22) Date de dépôt: 06.06.2012
(51) Int. Cl.: G01N 27/12, G01N 33/00, C22C 5/04, C22C 30/00

(54) **CAPTEUR D'HYDROGENE A COUCHE ACTIVE ET PROCEDE DE FABRICATION DE CAPTEURS D'HYDROGENE**
WASSERSTOFFSENSOR MIT EINER AKTIVEN SCHICHT UND VERFAHREN ZUR HERSTELLUNG VON WASSERSTOFFSENSOREN
HYDROGEN SENSOR WITH AN ACTIVE LAYER AND METHOD OF MANUFACTURING HYDROGEN SENSORS

(30) Priorité: 15.07.2011 CH 1192011
(43) Date de publication de la demande: 21.05.2014
(73) Titulaire: The Swatch Group Research and Development Ltd., 2074 Marin (CH)
(72) Inventeur: KLAUS, Yvon, CH-1233 Bernex (CH); KOLLER, Edmond, CH-1202 Genève (CH); RAPIN, Jean-Philippe, F-74930 Esery (FR); STALDER, Michael, CH-2503 Bienne (CH)
(74) Mandataire: Surmely, Gérard
(86) Numéro de dépôt international: PCT/EP2012/060755
(87) Numéro de publication internationale: WO 2013/010721

(56) Documents cités:
- US-A1- 2004 093 928
- YVON ET AL: "LaMg2PdH7, a new complex metal hydride containing tetrahedral [PdH4]<4-> anions", JOURNAL OF ALLOYS AND COMPOUNDS, vol. 446-447, 11 octobre 2007 (2007-10-11) , pages 34-38, XP022294029, ELSEVIER SEQUOIA, LAUSANNE, CH ISSN: 0925-8388, DOI: 10.1016/J.JALLCOM.2006.11.209
- K. YVON ET AL: "Hydrogenation-Induced Insulating State in the Intermetallic Compound LaMg2Ni", PHYSICAL REVIEW LETTERS, vol. 94, no. 6, 1 février 2005 (2005-02-01), pages 066403-1, XP055037612, ISSN: 0031-9007, DOI: 10.1103/PhysRevLett.94.066403
- Anonymous: "Hydrogen Sensor entry in Wikipedia", Wikipedia, the free encyclopedia , 24 décembre 2008 (2008-12-24), XP002683065, Extrait de l'Internet: URL:http://en.wikipedia.org/wiki/Hydrogen_ sensor [extrait le 2012-09-07]

## Description

### DOMAINE TECHNIQUE DE L'INVENTION

La présente invention concerne un capteur d'hydrogène ainsi qu'un détecteur d'hydrogène intégrant un tel capteur, ce détecteur permettant la détection d'hydrogène et/ou la mesure de la concentration d'hydrogène dans un milieu dans lequel est disposé le capteur. Elle concerne également un procédé de fabrication de capteurs d'hydrogène.

### ETAT DE LA TECHNIQUE ANTERIEURE

Les détecteurs et capteurs d'hydrogène connus proposant des caractéristiques pertinentes sont des produits coûteux à fabriquer et présentent de ce fait un intérêt restreint pour les utilisations sur des marchés de masse. Les solutions proposant des technologies moins coûteuses entraînent des limitations importantes des caractéristiques, par exemple au niveau des gammes de concentrations considérées pouvant être mesurées et/ou détectées et/ou de la possibilité de mesurer ou de détecter l'hydrogène parmi plusieurs autres gaz, etc.

Pour pallier ces différents inconvénients, l'invention prévoit différents moyens techniques.

### EXPOSE DE L'INVENTION

Tout d'abord, un premier objet de l'invention consiste à fournir un capteur d'hydrogène ainsi qu'un détecteur intégrant un tel capteur qui présentent des prix de revient avantageux par rapport aux capteurs et aux détecteurs d'hydrogène existants.

Un autre objet de l'invention consiste à fournir un capteur et un détecteur d'hydrogène permettant de mesurer et de détecter des concentrations d'hydrogène dans une gamme de concentrations allant de 0 à 100 % de H₂.

Encore un autre objet de l'invention consiste à fournir un capteur et un détecteur d'hydrogène qui soient précis et fiables.

Enfin, encore un autre objet de l'invention consiste à fournir un capteur et un détecteur permettant de mesurer et de détecter l'hydrogène présent parmi un mélange d'autres gaz.

A cet effet, l'invention concerne un capteur d'hydrogène comprenant un substrat sur lequel est déposée une couche active de matériau comprenant un premier élément sélectionné parmi la famille des terres rares ou une combinaison d'au moins deux éléments de la famille des terres rares, un deuxième élément sélectionné parmi les métaux du groupe platine (MGP) ou une combinaison d'au moins deux éléments métaux du groupe platine (MGP) (ou des combinaisons de ces derniers), et un troisième élément sélectionné parmi la famille des métaux alcalino-terreux ou une combinaison d'au moins deux éléments de la famille des métaux alcalino-terreux permettant l'absorption et la désorption d'hydrogène à température ambiante. La couche active comporte une épaisseur de 1 nm à 0,1 mm, et plus particulièrement de 5 nm à 10'000 nm et encore plus particulièrement de 50 nm à 1'500 nm.

La résistance électrique de la couche active change de façon sensiblement proportionnelle à la concentration d'hydrogène en contact avec cette couche active, ce qui rend possible l'utilisation de cette couche en tant que capteur d'hydrogène. En outre, la résistance électrique de la couche active dépend principalement de la pression partielle d'hydrogène et non pas de la pression totale de gaz du milieu dans lequel le capteur est disposé. Par ailleurs, la réaction au niveau de la couche active est réversible et la couche active reste intacte même après plusieurs cycles d'absorption et de désorption d'hydrogène. Les couches actives sont utilisables et efficaces sur une vaste gamme de concentrations d'hydrogène à la température ambiante.

La réponse des capteurs est très rapide, avec une bonne reproductibilité du signal. L'utilisation d'un composé métallique tel que le LaMg₂Pd permet de réaliser des couches actives utilisables en tant que capteur. Dans une variante, les couches actives sont utilisées à des fins de mesure de l'hydrogène.

Des mesures ont permis de démontrer qu'une transition métal-isolant induite par l'hydrogène se produit à température sensiblement ambiante et à des pressions d'hydrogène sensiblement peu élevées, entre une phase partiellement chargée de composition LaMg₂PdH₃ et une phase entièrement chargée de composition LaMg₂PdH₇.

De manière préférentielle, le premier élément de la couche active est sélectionné parmi la famille des lanthanides. Encore plus préférentiellement, le premier élément de la couche active est sélectionné parmi le sous groupe comprenant le Lanthane, le Cérium, le Praséodyme, le Néodyme, le Samarium, l'Europium ou une combinaison d'au moins deux éléments des métaux de ce sous groupe.

De manière préférentielle, le deuxième élément de la couche active est sélectionné parmi le palladium et le platine, et encore plus préférentiellement parmi le palladium et le platine.

De manière préférentielle, le troisième élément de la couche active est sélectionné parmi le magnésium et le calcium. De manière encore plus préférentielle, le troisième élément de la couche active est constitué de magnésium.

De manière avantageuse, le substrat comprend un matériau sélectionné parmi le titanate de strontium (SrTiO₃), le verre (borosilicate), le silicium, le mica. Bien entendu tout autre matériau adapté au dépôt d'une couche métallique active par des procédés de dépôt sous vide classiques peut être envisagé comme substrat.

Dans un mode de réalisation avantageux, la couche active est recouverte d'une couche de recouvrement, constituée de palladium et/ou de platine.

Selon un autre aspect, l'invention concerne un détecteur d'hydrogène comprenant le capteur d'hydrogène décrit ci-dessus et un module électronique de mesure relié audit capteur, ledit module électronique étant agencé pour mesurer un paramètre d'état dudit capteur et étant susceptible de fournir en sortie un signal électrique de mesure représentatif d'une concentration d'hydrogène d'un milieu dans lequel le capteur est disposé.

Selon une caractéristique avantageuse du détecteur, le module électronique de mesure est agencé pour mesurer la résistance électrique de la couche active et fournir un signal représentatif de ladite résistance électrique par exemple à un système de traitement ou un système d'enregistrement, puis à un dispositif d'affichage.

Selon un mode particulier de réalisation du détecteur d'hydrogène selon l'invention, le module électronique de mesure comprend en outre un comparateur relié audit capteur, et le signal électrique de mesure représentatif de la concentration d'hydrogène mesuré par le module à partir dudit capteur est fourni en entrée dudit comparateur, ledit comparateur étant agencé pour fournir en sortie un signal de détection fonction de la comparaison dudit signal d'entrée avec au moins un signal de seuil prédéterminé et de préférence, le module électronique de mesure est agencé pour que le au moins un signal de seuil prédéterminé soit programmable.

L'invention prévoit par ailleurs un procédé de fabrication du capteur d'hydrogène décrit ci-dessus, comprenant les étapes suivantes:
- fournir un substrat adapté pour application d'une couche métallique active;
- déposer une couche métallique active sur ledit substrat, la couche métallique active étant constituée d'un matériau dont un premier élément est sélectionné parmi la famille des terres rares ou une combinaison d'au moins deux éléments de la famille des terres rares, un deuxième élément est sélectionné parmi les métaux du groupe platine (MGP) ou une combinaison d'au moins deux éléments des métaux du groupe platine (MGP), et un troisième élément est sélectionné parmi la famille des métaux alcalino-terreux ou une combinaison d'au moins deux éléments de la famille des métaux alcalino-terreux.

Le procédé utilisé est particulièrement simple à mettre en oeuvre, donc peu cher, offre une bonne reproductibilité, et peut être réalisé avec une installation compacte.

Le dépôt de la couche active est avantageusement effectué par un procédé de dépôt physique en phase vapeur (PVD) et de préférence par pulvérisation cathodique. Dans ce dernier cas, l'atmosphère de pulvérisation cathodique est principalement constituée d'argon. Toujours dans ce même cas, avant le dépôt, on chauffe le substrat à une température d'au moins 250°C, et de préférence à une température de 300 °C.

Dans une variante de réalisation, le dépôt de la couche active est effectué par évaporation sous vide.

L'invention concerne également l'utilisation d'un composé sélectionné parmi les composés suivants : LaMg₂Pd, LaMg₂Pt, Sc(ₓ)La(₁₋ₓ)Mg₂Pd, La(1-x)Ce(x)Mg2Pd, LaMg(2-x)Ca(x)Pd, LaMg2Pd(1-x)Pt(x), 0<x<1 pour la réalisation d'une couche active pour un capteur d'hydrogène pour l'évaluation de la présence ou de la concentration d'hydrogène en contact avec ladite couche active. De préférence le LaMg₂Pd sera utilisé pour la réalisation d'une couche active pour un capteur d'hydrogène pour l'évaluation de la présence ou de la concentration d'hydrogène en contact avec ladite couche active. L'invention concerne également l'utilisation d'un composé susmentionné, et selon laquelle ladite couche active forme avec l'hydrogène du milieu dans lequel est inséré ledit capteur un hydrure de composition variable selon la concentration en hydrogène dudit milieu, chaque composition particulière dudit hydrure correspondant à une valeur particulière de la résistance électrique de ladite couche active.

### DESCRIPTION DES FIGURES

D'autres caractéristiques et avantages du capteur et du détecteur d'hydrogène selon l'invention ressortiront également de la description détaillée suivante de modes de réalisation du capteur et du détecteur de l'invention, cette description étant effectuée à l'aide des figures annexées, données à titre d'exemples nullement limitatifs et dans lesquels :
- la Figure 1 est un graphique présentant l'évolution de la résistance électrique relative en fonction du temps pour un capteur selon l'invention soumis à une série de cycles d'absorption et de désorption sous hydrogène pur à température ambiante (25°C) ;
- la Figure 2a est un graphique montrant l'évolution de la résistance électrique absolue pour un capteur selon l'invention en fonction du temps pour trois exemples de concentrations d'hydrogène ;
- la Figure 2b est un graphique montrant l'évolution de la résistance électrique relative pour un capteur selon l'invention soumis à des pressions partielles allant jusqu'à 300 mbar d'hydrogène et des pressions partielles d'argon allant de 700 à 900 mbar ;
- la Figure 3a est une représentation schématique en coupe d'un capteur d'hydrogène selon l'invention ;
- la Figure 3b est une représentation schématique d'un mode de réalisation d'un détecteur d'hydrogène intégrant un capteur d'hydrogène selon l'invention.

### DESCRIPTION DETAILLEE DE L'INVENTION

On entend par métaux du groupe platine (MGP) : ruthénium, rhodium, osmium, iridium, platine, rhénium, palladium, ou une combinaison de ces éléments.

On entend par métal alcalino-terreux : magnésium, calcium, strontium, baryum, ou une combinaison de ces éléments.

On entend par matériaux de la famille terres rares: Lanthane, Cérium, Praséodyme, Néodyme, Samarium, Europium, Gadolinium, Terbium, Dysprosium, Holmium, Erbium, Thulium, Ytterbium, Lutécium, ou un des éléments Yttrium ou Scandium ou une combinaison de ces éléments.

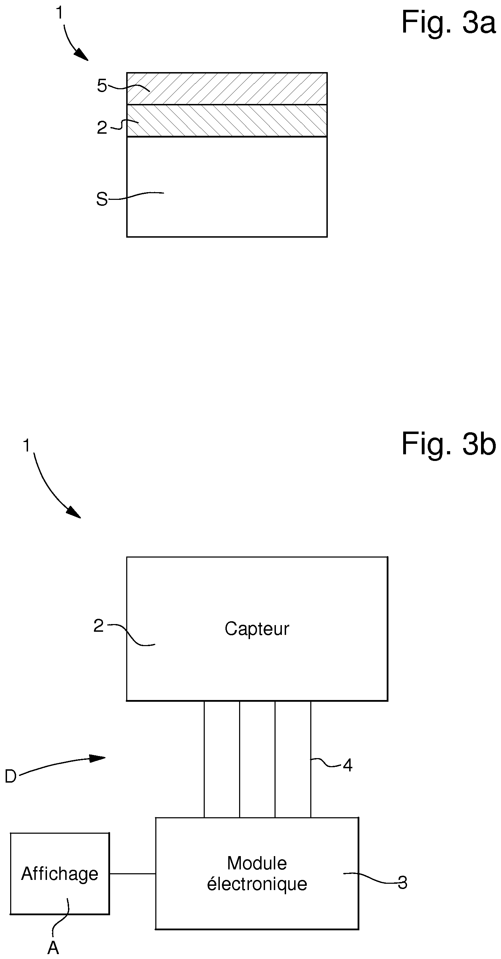

La figure 3a montre un exemple d'un capteur 1 selon l'invention. Il comprend un substrat S, par exemple sous la forme d'un film ayant par exemple une épaisseur de 100 microns, sur lequel une couche mince active 2 a été déposée. Le capteur 1 est relié par sa couche 2 à un module électronique 3 agencé notamment pour mesurer un paramètre d'état du capteur, en l'occurrence la résistance électrique de la couche 2, via quatre (ou deux en variante) liaisons électriques 4 pour former un détecteur D visible à la figure 3b. Dans une variante de réalisation, le module électronique 3 et le substrat S sont intégrés pour ne former qu'un seul élément. Le module 3 est conçu pour mesurer la résistance électrique de la couche active, par exemple par la méthode des quatre pointes ou par toute autre méthode appropriée et fournir un signal de mesure représentatif de la concentration en hydrogène du milieu dans lequel est placé le capteur. Ce signal de mesure est traité par un circuit électronique puis la valeur de concentration mesurée est ensuite affichée sur un écran d'affichage A.

Selon une variante de réalisation, le module électronique de mesure 3 peut être agencé pour fournir un signal de détection de la présence d'hydrogène. Dans ce cas, le module électronique de mesure comprend en outre un comparateur (non représenté) et le signal électrique de mesure représentatif d'une concentration d'hydrogène mesuré par le module électronique de mesure à partir dudit capteur est fourni en entrée au comparateur, ce dernier étant agencé pour fournir en sortie un signal de détection fonction de la comparaison dudit signal d'entrée avec au moins un signal de seuil prédéterminé. Le module électronique est avantageusement agencé pour que le au moins un signal de seuil prédéterminé soit programmable selon un ou plusieurs niveaux de concentration d'hydrogène mesuré. Selon les besoins, le circuit pourra être agencé pour délivrer un signal d'alarme, par exemple sonore ou visuel dans le cas d'un dépassement d'un ou des seuils de concentration prédéterminés.

La couche active 2 est constituée d'un matériau comportant trois éléments : un premier élément est sélectionné parmi la famille des terres rares ou une combinaison d'au moins deux éléments de la famille des terres rares, un deuxième élément est sélectionné parmi les métaux du groupe platine (MGP) ou une combinaison d'au moins deux éléments de la famille des métaux du groupe platine (MGP) et un troisième élément est sélectionné parmi la famille des métaux alcalino-terreux ou une combinaison d'au moins deux éléments de la famille des métaux alcalino-terreux.

Voici quelques exemples, fournis à titre non limitatif, de couches actives sensibles à l'hydrogène : LaMg₂Pd, LaMg₂Pt, Sc(ₓ)La(₁₋ₓ)Mg₂Pd, La(1-x)Ce(x)Mg2Pd, LaMg(2-x)Ca(x)Pd, LaMg2Pd(1-x)Pt(x), 0<x<1, etc.

Divers types de substrats peuvent être utilisés, comme par exemple un substrat constitué de titanate de strontium (SrTiO₃), de verre (borosilicate), de silicium, de mica, ou autres dès lors qu'ils sont adaptés au dépôt d'une couche active par des procédés de dépôt sous vide classiques.

Les gammes d'épaisseurs de la couche active 2 sont de préférence de : 1nm à 0.1mm, plus particulièrement de 5nm à 10'000 nm et encore plus particulièrement de 50nm à 1'500nm. Pour le substrat, il n'y a en principe aucune limitation d'épaisseur. En pratique, il est utile de considérer une épaisseur minimale de 1 micron.

Pour produire la couche active 2, un procédé particulièrement avantageux est prévu. Ainsi, le dépôt d'une couche mince est effectué par dépôt physique en phase vapeur (le terme anglais PVD « Physical Vapor Déposition » est couramment utilisé) par exemple par pulvérisation cathodique ou par évaporation, ou autre approche de dépôt physique en phase vapeur. On notera que des dépôts du type CVD ou ALD ou encore l'implantation ionique sont également envisageables.

Selon un exemple de réalisation, un substrat approprié pour déposer la couche mince métallique 2, comme par exemple un substrat de SrTiO₃, est chauffé à 300 °C, et est placé dans une atmosphère d'argon à une pression de 10⁻⁴ bars.

Une couche active d'environ 120 nm est obtenue après quelques minutes en utilisant un embout de pulvérisation d'une puissance de 10 Watts. Aucun autre traitement thermique n'est requis pour traiter la couche ainsi obtenue.

Les couches actives ainsi produites sont ensuite pré-chargées en hydrogène. Pour ce faire, on place le capteur dans un milieu contenant de l'hydrogène jusqu'à ce que la couche active soit saturée en H₂.

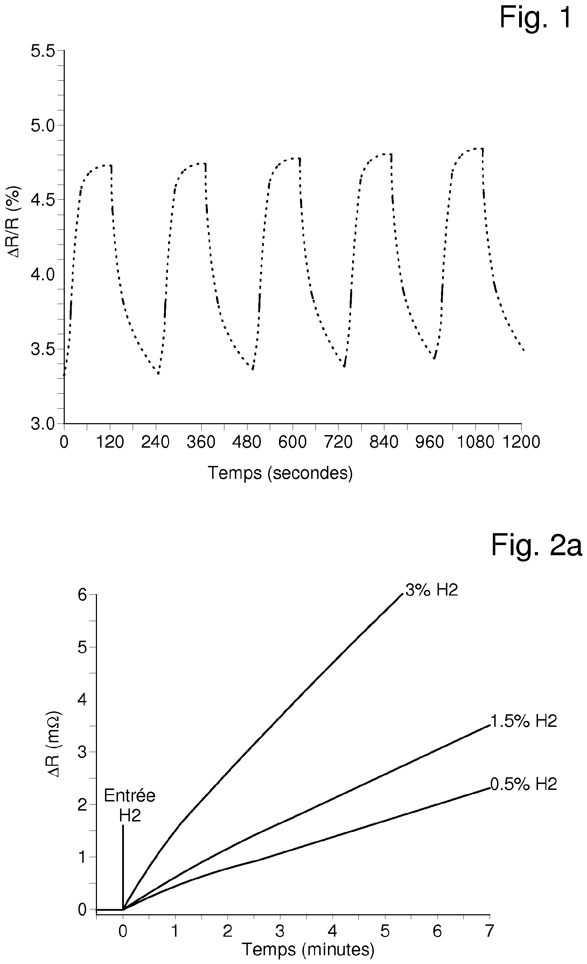

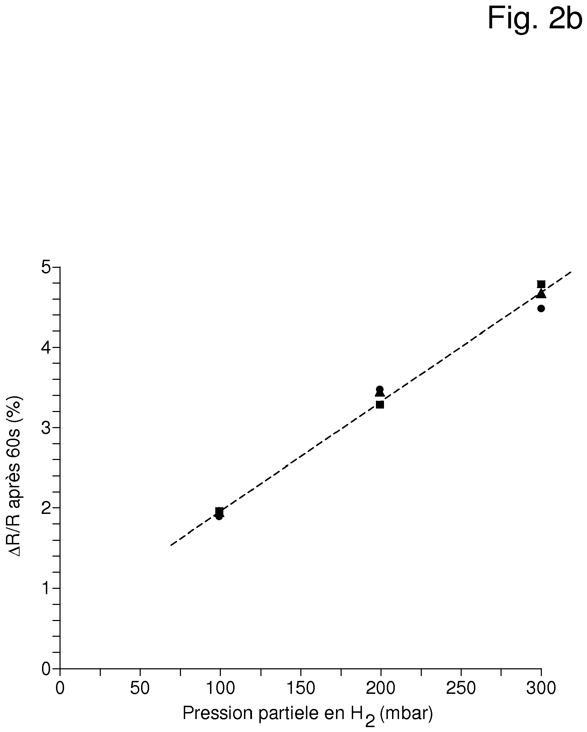

Les graphiques des figures 1 et 2 ont été produits à partir d'un alliage ternaire LaMg₂Pd. Cette couche active doit être pré-chargée en hydrogène pour arriver à l'hydrure LaMg₂PdH₃, avant l'utilisation comme capteur d'hydrogène selon l'invention.

La Figure 1 montre un exemple de substrat avec une couche active pré-chargée soumis à une série de cycles d'absorption et de désorption sous hydrogène pur à température ambiante (25°C). La résistance électrique de la couche active est mesurée par une méthode de mesure connue, dans ce cas la méthode des quatre pointes. La valeur de R correspond à la résistance électrique de l'alliage LaMg₂PdH₃ (composition de la couche active en l'absence d'hydrogène). Les cycles sont basés sur l'augmentation de la pression jusqu'à 1 bar pendant 2 minutes et la diminution de la pression jusqu'au vide pendant 2 minutes, tel que montré sur la figure. Ainsi, au temps t=0 on introduit 1 bar d'hydrogène dans la chambre du capteur (sous vide), à t=2 min on évacue de nouveau la chambre, à t=4 min on réintroduit 1 bar d'hydrogène, etc. On observe que la résistance électrique augmente d'environ 1,5 % pendant les phases d'absorption et diminue d'une valeur similaire lors de la désorption. La couche active produite permet une réponse rapide et une bonne reproductibilité du signal. Sur une échelle de quelques minutes, la dérive est sensiblement faible, tandis que sur une échelle de quelques heures la dérive est sensiblement nulle. Des analyses à partir de profils obtenus à l'aide de rayons X avant et après l'hydrogénation ont permis de confirmer la présence de la phase active LaMg₂Pd, de la phase intermédiaire partiellement chargée LaMg₂PdH₃, de la phase entièrement chargée LaMg₂PdH₇ et d'oxyde de lanthane La₂O₃.

La figure 2a illustre des mesures effectuées avec une couche active 2 soumise à diverses concentrations d'hydrogène (0.5%, 1.5% et 3%) ajoutées à l'atmosphère d'argon. La figure 2a montre que la résistance électrique augmente immédiatement pour toutes les concentrations. Il est à noter que la concentration la plus faible (0,5 %) permet de satisfaire les exigences d'un signal facilement identifiable, permettant de réaliser un capteur d'hydrogène à faible concentration. La demanderesse a remarqué que la couche active ne présente pas de dégradation du signal lors de cycles prolongés.

La figure 2b illustre les résultats de mesures effectuées à l'aide de trois capteurs selon l'invention, avec des pressions partielles allant jusqu'à 300 mbar d'hydrogène et des pressions partielles d'argon allant de 700 à 900 mbar. La figure montre la variation de résistance électrique relative (en %, après 60 secondes) pour une couche active de LaMg2Pd sur trois substrats distincts (symbolisés respectivement par des carrés, triangles et des ronds à la figure 2b), pour des pressions partielles variant entre 100 et 300 mbar, le reste du mélange étant constitué d'argon, pour une pression totale de 1 bar. On constate que la résistance électrique relative augmente de façon régulière et significative avec une excellente reproductibilité. Le capteur peut donc fournir des valeurs de pressions partielles d'hydrogène de façon fiable et précise, même pour des concentrations aussi importantes que 20% ou 30% d'hydrogène. Par ailleurs, des essais effectués avec de l'air au lieu de l'argon ont permis l'obtention de résultats similaires.

En présence d'hydrogène dans l'atmosphère, cet hydrogène est absorbé par la couche active 2. Cette absorption correspond à une réaction de conversion du LaMg₂PdH₃ en LaMg₂PdH₇. Ces deux hydrures ayant des résistivités électriques différentes, la résistance électrique mesurée dépend du taux de LaMg₂PdH₃ converti en LaMg₂PdH₇, qui dépend à son tour de la concentration d'hydrogène.

### Variante avec couche de recouvrement

Selon ce premier mode de réalisation, dans des concentrations d'hydrogène élevées, une fois que tout le LaMg₂PdH₃ a été converti en LaMg₂PdH₇, le capteur est saturé et la résistance électrique de la couche active n'augmente plus avec la concentration d'hydrogène. Dans ces cas de concentrations d'hydrogène élevées, le dispositif n'est plus capable d'assurer une fonction de mesure et se trouve restreint à une fonction de détection.

Pour pallier cet inconvénient, il est prévu de recouvrir la couche active 2 (par exemple une couche de LaMg₂Pd) par une couche 5 (figure 3b) mince , typiquement de l'ordre de 4 nm) de palladium et/ou de platine. Avec une telle couche de recouvrement 5, la couche active réagit différemment. En effet, au lieu d'une réaction de conversion telle que décrite précédemment, cet ensemble entraîne un nouvel équilibre entre le contenu d'hydrogène x dans la couche active, ici l'hydrure LaMg₂PdHₓ et la pression d'hydrogène. Grâce à cet équilibre, le capteur n'atteint pas de saturation complète tout en montrant une cinétique plus rapide et un temps de récupération plus court. Le domaine d'application s'étend ainsi du détecteur aux capteurs. Dans une variante de réalisation, la couche de palladium est remplacée par une couche similaire d'un alliage de Pt et Pd avec éventuelle du Nickel.

La différence de réactivité en fonction de la pression d'hydrogène peut s'expliquer par la capacité du palladium à dissocier la molécule d'hydrogène H₂ en hydrogène monoatomique beaucoup plus réactif. Cet effet explique également un autre aspect très positif de la couche de palladium. En présence d'oxygène dans l'atmosphère, l'hydrogène est résorbé beaucoup plus rapidement de la couche, parce que l'hydrogène monoatomique réagit sur la surface de palladium pour former de l'eau ou de l'hydrogène moléculaire. Le temps de réponse du capteur lorsque la concentration d'hydrogène baisse est ainsi nettement amélioré.

## Revendications

1. Capteur d'hydrogène comprenant un substrat (S) sur lequel est déposée une couche active (2) de matériau présentant une composition comprenant un premier élément sélectionné parmi la famille des terres rares ou une combinaison d'au moins deux éléments de la famille des terres rares, un deuxième élément sélectionné parmi les métaux du groupe platine (MGP) ou une combinaison d'au moins deux éléments des métaux du groupe platine (MGP), et un troisième élément sélectionné parmi la famille des métaux alcalino-terreux ou une combinaison d'au moins deux éléments de la famille des alcalino-terreux permettant l'absorption et la désorption d'hydrogène à température ambiante et dans lequel la couche active comporte une épaisseur de 1nm à 0,1 mm, et plus particulièrement de 5nm à 10'000 nm et encore plus particulièrement de 50 nm à 1'500 nm.

2. Capteur d'hydrogène selon la revendication 1, dans lequel le troisième élément de la couche active est sélectionné parmi le magnésium ou le calcium ou une combinaison de ces éléments.

3. Capteur d'hydrogène selon l'une des revendications 1 ou 2, dans lequel le deuxième élément de la couche active est sélectionné parmi le palladium ou le platine ou une combinaison de ces éléments.

4. Capteur d'hydrogène selon l'une des revendications 1 à 3, dans lequel le premier élément de la couche active est sélectionné parmi la famille des terres rares ou une combinaison d'au moins deux éléments du groupe des terres rares.

5. Capteur d'hydrogène selon l'une des revendications 1 à 3 dans lequel le premier élément de la couche active est sélectionné parmi le sous groupe comprenant le Lanthane, le Cérium, le Praséodyme, le Néodyme, le Samarium, l'Europium ou une combinaison d'au moins deux éléments des métaux de ce sous groupe.

6. Capteur d'hydrogène selon l'une des revendications précédentes, dans lequel le substrat comprend un matériau sélectionné parmi le titanate de strontium, le verre, le silicium, le mica.

7. Capteur d'hydrogène selon l'une des revendications précédentes, dans lequel la couche active est constituée d'un matériau sélectionné parmi les suivants : LaMg₂Pd, LaMg₂Pt, Sc₍ₓ₎La₍₁₋ₓ)Mg₂Pd, La(1-x)Ce(x)Mg2Pd, LaMg(2-x)Ca(x)Pd, LaMg2Pd(1-x)Pt(x), 0<x<1.

8. Capteur d'hydrogène selon l'une des revendications précédentes dans lequel la couche active est recouverte d'une couche de recouvrement, comprenant du palladium et/ou du platine.

9. Détecteur d'hydrogène comprenant un capteur d'hydrogène et un module électronique de mesure (3) relié audit capteur, ledit module électronique étant agencé pour mesurer un paramètre d'état dudit capteur et étant susceptible de fournir en sortie un signal électrique de mesure représentatif d'une concentration d'hydrogène dans lequel le capteur est disposé, **caractérisé en ce que** ledit capteur d'hydrogène est un capteur selon l'une quelconque des revendications 1 à 8.

10. Détecteur d'hydrogène selon la revendication 9, **caractérisé en ce que** le module électronique de mesure (3) est agencé pour mesurer la résistance électrique de la couche active et fournir un signal représentatif de ladite résistance électrique à un système de traitement ou un système d'enregistrement puis à un dispositif d'affichage

11. Détecteur d'hydrogène selon la revendication 9 ou 10 caractérisé en que le module électronique de mesure (3) comprend en outre un comparateur relié audit capteur, et le signal électrique de mesure représentatif de la concentration d'hydrogène mesuré par le module à partir dudit capteur est fourni en entrée dudit comparateur, ledit comparateur étant agencé pour fournir en sortie un signal de détection fonction de la comparaison dudit signal d'entrée avec au moins un signal de seuil prédéterminé.

12. Détecteur d'hydrogène selon la revendication 11, **caractérisé en ce que** le module électronique est agencé pour que le au moins un signal de seuil prédéterminé soit programmable.

13. Procédé de fabrication d'un capteur d'hydrogène selon l'une des revendications précédentes, comprenant les étapes suivantes:
- fournir un substrat adapté pour application d'une couche métallique active;
- déposer une couche métallique active sur ledit substrat, la couche métallique active étant constituée d'un matériau dont un premier élément est sélectionné parmi la famille des terres rares ou une combinaison d'au moins deux éléments de la famille des terres rares, un deuxième élément est sélectionné parmi les métaux du groupe platine (MGP) ou une combinaison d'au moins deux éléments de la famille du groupe platine et un troisième élément est sélectionné parmi la famille des métaux alcalino-terreux ou une combinaison d'au moins deux éléments de la famille des métaux alcalino-terreux.

14. Procédé de fabrication de capteurs d'hydrogène selon la revendication 13, dans lequel le dépôt de la couche active est effectué par dépôt physique en phase vapeur.

15. Procédé de fabrication de capteurs d'hydrogène selon la revendication 14, dans lequel le dépôt de la couche active est effectué par évaporation ou pulvérisation cathodique.

16. Procédé de fabrication de capteurs d'hydrogène selon l'une des revendications 13 à 14, dans lequel la couche active est pré-chargée en hydrogène de manière à former un premier hydrure, avant son utilisation comme capteur.

17. Utilisation d'un composé sélectionné parmi les composés suivants: LaMg₂Pd, LaMg₂Pt, Sc₍ₓ₎La₍₁₋ₓ₎Mg₂Pd, La(1-x)Ce(x)Mg2Pd, LaMg(2-x)Ca(x)Pd, LaMg2Pd(1-x)Pt(x), 0<x<1 pour la réalisation d'une couche active d'une épaisseur de 1 nm à 0,1 mm, et plus particulièrement de 5 nm à 10'000 nm et encore plus particulièrement de 50 nm à 1'500 nm pour un capteur d'hydrogène pour l'évaluation de la présence ou de la concentration d'hydrogène en contact avec ladite couche active.

18. Utilisation du LaMg₂Pd pour la réalisation d'une couche active d'une épaisseur de 1 nm à 0,1 mm, et plus particulièrement de 5 nm à 10'000 nm et encore plus particulièrement de 50 nm à 1'500 nm pour un capteur d'hydrogène pour l'évaluation de la présence ou de la concentration d'hydrogène en contact avec ladite couche active.

19. Utilisation dudit composé selon la revendication 17 ou 18 **caractérisée en ce que** ladite couche active forme avec l'hydrogène du milieu dans lequel est inséré ledit capteur un hydrure de composition variable selon la concentration en hydrogène dudit milieu, chaque composition particulière dudit hydrure correspondant à une valeur particulière de la résistance électrique de ladite couche active.

## Patentansprüche

1. Wasserstoffsensor, umfassend ein Substrat (S), auf dem eine aktive Materialschicht (2) abgeschieden ist, mit einer Zusammensetzung, die ein erstes Element, das aus der Familie der seltenen Erden oder aus einer Kombination aus mindestens zwei Elementen der Familie der seltenen Erden ausgewählt ist, ein zweites Element, das unter den Platingruppenmetallen (PGM) oder einer Kombination aus mindestens zwei Elementen der Platingruppenmetalle (PGM) ausgewählt ist, und ein drittes Element umfasst, das aus der Familie der Erdalkalimetalle oder aus einer Kombination aus mindestens zwei Elementen der Familie der Erdalkalimetalle ausgewählt ist, das die Absorption und die Desorption von Wasserstoff bei Umgebungstemperatur ermöglicht, wobei die aktive Schicht eine Dicke im Bereich von 1 nm bis 0,1 mm, bevorzugt von 5 nm bis 10000 nm, und besonders bevorzugt von 50 nm bis 1500 nm aufweist.

2. Wasserstoffsensor nach Anspruch 1, wobei das dritte Element der aktiven Schicht ausgewählt ist aus Magnesium oder Calcium oder einer Kombination dieser Elemente.

3. Wasserstoffsensor nach einem der Ansprüche 1 oder 2, wobei das zweite Element der aktiven Schicht ausgewählt ist aus Palladium oder Platin oder einer Kombination dieser Elemente.

4. Wasserstoffsensor nach einem der Ansprüche 1 bis 3, wobei das erste Element der aktiven Schicht ausgewählt ist aus der Familie der seltenen Erden oder einer Kombination aus mindestens zwei Elementen der Gruppe der seltenen Erden.

5. Wasserstoffsensor nach einem der Ansprüche 1 bis 3, wobei das erste Element der aktiven Schicht aus der Untergruppe gewählt ist, die Lanthan, Cer, Praseodym, Neodym, Samarium, Europium oder eine Kombination aus mindestens zwei Elementen der Metalle dieser Untergruppe enthält.

6. Wasserstoffsensor nach einem der vorhergehenden Ansprüche, wobei das Substrat ein Material enthält, das ausgewählt ist aus Strontiumtitanat, Glas, Silicium und Glimmer.

7. Wasserstoffsensor nach einem der vorhergehenden Ansprüche, wobei die aktive Schicht aus einem Material gebildet ist, das ausgewählt ist aus den folgenden Verbindungen: LaMg₂Pd, LaMg₂Pt, Sc₍ₓ₎La₍₁₋ₓ₎Mg₂Pd, La₍₁₋ₓ₎Ce₍ₓ₎Mg₂Pd, LaMg₍₂₋ₓ₎Ca₍ₓ₎Pd, LaMg₂Pd₍₁₋ₓ₎Pt₍ₓ₎, 0 < x < 1.

8. Wasserstoffsensor nach einem der vorhergehenden Ansprüche, wobei die aktive Schicht mit einer Deckschicht beschichtet ist, die Palladium und/oder Platin enthält.

9. Wasserstoffdetektor, umfassend einen Wasserstoffsensor und ein mit dem Sensor verbundenes elektronisches Messmodul (3), wobei das elektronische Modul zum Messen eines Zustandsparameters des Sensors angeordnet ist, und geeignet ist, am Ausgang ein elektrisches Messsignal auszugeben, das eine Wasserstoffkonzentration, in der der Sensor angeordnet ist, repräsentiert, **dadurch gekennzeichnet, dass** der Wasserstoffsensor ein Sensor nach einem der Ansprüche 1 bis 8 ist.

10. Wasserstoffdetektor nach Anspruch 9, **dadurch gekennzeichnet, dass** das elektronische Messmodul (3) dafür ausgelegt ist, den elektrischen Widerstand der aktiven Schicht zu messen und ein Signal, das den elektrischen Widerstand repräsentiert, an ein Verarbeitungssystem oder an ein Aufzeichnungssystem und dann an eine Anzeigevorrichtung zu übertragen.

11. Wasserstoffdetektor nach Anspruch 9 oder 10, **dadurch gekennzeichnet, dass** das elektronische Messmodul (3) weiterhin einen Komparator umfasst, der mit dem Sensor verbunden ist, und das elektrische Messsignal, das die Wasserstoffkonzentration repräsentiert und durch das Modul vom Sensor gemessen wird, an den Eingang des Komparators übertragen wird, wobei der Komparator dafür ausgelegt ist, am Ausgang ein Detektionssignal auszugeben, das eine Funktion des Vergleichs des Eingangssignals mit mindestens einem vorbestimmten Schwellensignal ist.

12. Wasserstoffdetektor nach Anspruch 11, **dadurch gekennzeichnet, dass** das elektronische Modul dafür ausgelegt ist, dass das mindestens eine vorbestimmte Schwellensignal programmierbar ist.

13. Verfahren zur Herstellung eines Wasserstoffsensors nach einem der vorhergehenden Ansprüche, umfassend die folgenden Schritte:
- Bereitstellen eines Substrats, das zum Aufbringen einer aktiven Metallschicht geeignet ist;
- Abscheiden einer aktiven Metallschicht auf dem Substrat, wobei die aktive Metallschicht aus einem Material gebildet ist, von dem ein erstes Element ausgewählt ist aus der Familie der seltenen Erden oder aus einer Kombination aus mindestens zwei Elementen der Familie der seltenen Erden, ein zweites Element ausgewählt ist unter den Platingruppenmetallen (PGM) oder aus einer Kombination aus mindestens zwei Elementen der Familie der Platingruppe, und ein drittes Element ausgewählt ist aus der Familie der Erdalkalimetalle oder einer Kombination aus mindestens zwei Elementen der Familie der Erdalkalimetalle.

14. Verfahren zur Herstellung von Wasserstoffsensoren nach Anspruch 13, wobei das Abscheiden der aktiven Schicht durch physikalische Dampfabscheidung erfolgt.

15. Verfahren zur Herstellung von Wasserstoffsensoren nach Anspruch 14, wobei das Abscheiden der aktiven Schicht durch Verdampfung oder Katodenzerstäubung erfolgt.

16. Verfahren zur Herstellung von Wasserstoffsensoren nach einem der Ansprüche 13 bis 14, wobei die aktive Schicht vorab mit Wasserstoff angereichert wird, derart, dass vor ihrer Verwendung als Sensor ein erstes Hydrid gebildet wird.

17. Verwendung einer Verbindung, die aus den folgenden Verbindungen ausgewählt ist: LaMg₂Pd, LaMg₂Pt, Sc₍ₓ₎La₍₁₋ₓ₎Mg₂Pd, La₍₁₋ₓ₎Ce₍ₓ₎Mg₂Pd, LaMg₍₂₋ₓ₎Ca₍ₓ₎Pd, LaMg₂Pd₍₁₋ₓ₎Pt₍ₓ₎, 0 < x < 1, zur Herstellung einer aktiven Schicht mit einer Dicke im Bereich von 1 nm bis 0,1 mm, bevorzugt von 5 nm bis 10000 nm, und besonders bevorzugt von 50 nm bis 1500 nm für einen Wasserstoffsensor zur Bewertung des Vorhandenseins oder der Konzentration von Wasserstoff, der mit der aktiven Schicht in Kontakt ist.

18. Verwendung von LaMg₂Pd zur Herstellung einer aktiven Schicht mit einer Dicke von 1 nm bis 0,1 mm, bevorzugt von 5 nm bis 10000 nm, und besonders bevorzugt von 50 nm bis 1500 nm für einen Wasserstoffsensor zur Bewertung des Vorhandenseins oder der Konzentration von Wasserstoff, der mit der aktiven Schicht in Kontakt ist.

19. Verwendung der Verbindung nach Anspruch 17 oder 18, **dadurch gekennzeichnet, dass** die aktive Schicht mit Wasserstoff des Mediums, in das der Sensor eingesetzt ist, je nach Konzentration des Wasserstoffs des Mediums ein Hydrid mit veränderlicher Zusammensetzung bildet, wobei jede bestimmte Zusammensetzung des Hydrids einem bestimmten Wert des elektrischen Widerstands der aktiven Schicht entspricht.

## Claims

1. Hydrogen sensor including a substrate (S) on which there is deposited an active layer (2) of material including a composition comprising a first element selected from the rare earth family or a combination of at least two elements from the rare earth family, a second element selected from the platinum group metals (PGMs) or a combination of at least two elements from the platinum group metals (PGMs), and a third element selected from the alkaline earth metal family or a combination of at least two elements from the alkaline earth metal family for the absorption and desorption of hydrogen at ambient temperature and wherein the active layer includes a thickness of 1nm to 0.1mm, and more specifically, from 5nm to 10000nm and even more specifically, from 50nm to 1500nm.

2. Hydrogen sensor according to claim 1, wherein the third element of the active layer is selected from among magnesium or calcium or a combination of these elements.

3. Hydrogen sensor according to claim 1 or 2, wherein the second element of the active layer is selected from among palladium or platinum or a combination of these elements.

4. Hydrogen sensor according to one of claims 1 to 3, wherein the first element of the active layer is selected from among the rare earth family or a combination of at least two elements from the rare earth group.

5. Hydrogen sensor according to one of claims 1 to 3, wherein the first element of the active layer is selected from the sub-group including lanthanum, cerium, praseodymium, neodymium, samarium, europium or a combination of at least two metal elements from this sub-group.

6. Hydrogen sensor according to one of the preceding claims, wherein the substrate includes a material selected from strontium titanate, glass, silicon, mica.

7. Hydrogen sensor according to one of the preceding claims, wherein the active layer is formed from a material selected from among the following: LaMg₂Pd, LaMg₂Pt, Sc₍ₓ₎La₍₁₋ₓ₎Mg₂Pd, La(1-x)Ce(x)Mg2Pd, LaMg(2-x)Ca(x)Pd, LaMg2Pd(1-x)Pt(x), 0<x<1, etc.

8. Hydrogen sensor according to one of the preceding claims, wherein the active layer is coated with a coating layer including palladium and/or platinum.

9. Hydrogen detector including a hydrogen sensor and an electronic measuring module (3) connected to said sensor, said electronic module being arranged to measure a state parameter of said sensor and being capable of delivering at output an electrical measurement signal representative of a hydrogen concentration in which the sensor is arranged, **characterised in that** said hydrogen sensor is a sensor according to any one of claims 1 to 8.

10. Hydrogen detector according to claim 9, **characterised in that** the electronic measuring module (3) is arranged to measure the electrical resistance of the active layer and to deliver a signal representative of said electrical resistance to a processing system or a storage system and then to a display device.

11. Hydrogen detector according to claim 9 or 10, **characterised in that** the electronic measuring module (3) further includes a comparator connected to said sensor, and the electronic measurement signal representative of a hydrogen concentration measured by the module from said sensor is delivered at input to said comparator, said comparator being arranged to deliver at output a detection signal as a function of the comparison of said input signal with at least one predetermined threshold signal.

12. Hydrogen detector according to the claim 11, **characterised in that** the electronic module is arranged so that said at least one predetermined threshold signal is programmable.

13. Method of manufacturing a hydrogen sensor according to one of the preceding claims, comprising the following steps:
- taking a substrate suitable for the application of an active metallic layer;
- depositing an active metallic layer on said substrate, the active metallic layer being formed of a material of which the first element is selected from the rare earth family, or a combination of at least two elements from the rare earth family, a second element is selected from the platinum group metals (PGMs), or a combination of at least two elements from the platinum group metals, and a third element is selected from the alkaline earth metal family or a combination of at least two elements from the alkaline earth metal family.

14. Method of manufacturing hydrogen sensors according to claim 13, wherein the active layer deposition is performed by physical vapour phase deposition.

15. Method of manufacturing hydrogen sensors according to claim 14, wherein the active layer deposition is performed by cathodic sputtering or evaporation.

16. Method of manufacturing hydrogen sensors according to one of claims 13 to 14, wherein the active layer is pre-charged with hydrogen so as to for a first hydride, prior to the use thereof as a sensor.

17. Use of a compound selected from among the following compounds: LaMg₂Pd, LaMg₂Pt, Sc₍ₓ₎La-₁₋₋ₓ₎Mg₂Pd, La₍₁₋ₓ₎Ce₍ₓ₎Mg₂Pd, LaMg(2-x)Ca(x)Pd, LaMg₂Pd(1--x)Pt(x), 0<x<1 to produce an active layer of a thickness of 1nm to 0.1mm, and more specifically from 5nm to 10000nm and even more specifically, from 50nm to 1500nm, for a hydrogen sensor for evaluating the presence or concentration of hydrogen in contact with said active layer.

18. Use of LaMg₂Pd to form an active layer of a thickness of 1nm to 0.1mm, and more specifically from 5nm to 10000nm and even more specifically, from 50nm to 1500nm, for a hydrogen sensor for evaluating the presence or concentration of hydrogen in contact with said active layer.

19. Use of said compound according to claim 17 or 18, **characterised in that** said active layer, with the hydrogen of the medium into which said sensor is inserted, forms a hydride of variable composition according to the concentration of hydrogen in said medium, each particular composition of said hydride corresponding to a particular electrical resistance value of said active layer.
